# EUROPEAN PATENT APPLICATION

(11) **EP 0 968 992 A1**
(43) Date of publication of application: **05.01.2000**
(21) Application number: 98303893.6
(22) Date of filing: 18.05.1998
(51) Int. Cl.: C07C 51/41, C07C 53/128

(54) **Process for producing lanthanide series metal corboxylates and their use for the polymerization of diene monomers**

(71) Applicant: Chi Mei Corporation, Jen-Te Hsiang, Tainan Hsien (TW)
(72) Inventor: Huang, Chen-Pao, An-Chang St., Tainan City (TW); Chu, Chih-Nan, Tainan City (TW)
(74) Representative: Woods, Geoffrey Corlett

(57) **Abstract**

A process for producing a compound of formula Ln(RCOO)₃ wherein Ln is a lanthanide series metal element of atomic number 57 to 71 and R is a C₁-C₁₉ hydrogen group, comprises reacting together:
(A) an oxide or carbonate of a lanthanide series metal Ln as defined above;
(B) a C₂-C₂₀ carboxylic acid;
(C) water; and
(D) an inert solvent
   at a temperature of from 0°C to 300°C, wherein the molar ratio of water to the lanthanide series metal is at least 5:1.

## Description

This invention relates to a process for the production of lanthanide series metal carboxylates and to the use of the lanthanide series metal carboxylates for the polymerization of diene monomers.

The production of lanthanide series metal carboxylates such as neodymium carboxylates is known. For example, U.S. Patent No. 4,520,177 discloses the production of neodymium versatate (Nd(versatate)₃) by reacting neodymium chloride solution with sodium versatate. The rough product of neodymium versatate thus obtained is then purified by extraction with toluene, followed by filtration of the toluene solution and drying. In addition, the production of neodymium trinaphthate has been disclosed by Changchun Institute of Applied Chemistry, Chinese Academic of Sciences (CIAC Changchun, Jilin, China). Their papers in the monograph of "Collected Papers on Synthetic Rubbers Prepared with Lanthanide Coordination Catalyst", were published by the Science Press, Beijing, 1980, pp 381-387. According to their disclosure, neodymium trinaphthate (Nd(Naph)₃) is produced by adding concentrated hydrogen chloride solution into neodymium oxide (Nd₂O₃) to produce neodymium chloride (NdCl₃) which is then mixed with ammonium hydroxide and naphthenic acid with solvent to obtain the neodymium trinaphthate solution. The solution thus obtained contains oil (solvent) phase and aqueous phase and is neutralized by ammonium hydroxide. After standing for the separation of the oil and aqueous phases, the separated oil phase is distilled to obtain the purified neodymium trinaphthate solution. In the purified neodymium trinaphthate solution, the molar ratio of free carboxylic acid (i.e. RCOOH which are not bonded to the neodymium metal) to neodymium is in the range of 0.45/1 to 0.8/1 and the moisture content is 78 to 425 ppm. The neodymium trinaphthate synthesized by said method can be used together with an alkyl aluminium chloride compound and an organo aluminium compound to provide a catalyst system for the polymerization of butadiene. In the catalyst system, the molar ratio of the free acid to the neodymium is in the range of 0.71/1 to 0.8/1 and the moisture content is in the range of 138 to 287 ppm. The catalyst system thus obtained is used for the polymerization of diene monomers with good conversion.

The preparations of neodymium carboxylates of US 4520177 and Changchun Institute still have some disadvantages. In US 4520177, it is necessary to proceed with the additional extraction and filtration step, and sometimes the metal carboxylate needs further purification to deprive the sodium ion. In the Changchun Institute method, the preparation is complicated. An additional NdCl₃ preparation step and a neutralization step are needed. Both preparations are not economical.

U.S. Patent 5,220,045 discloses the preparation of neodymium versatate (NdV₃) by introducing ammonia into a mixture of aqueous neodymium nitrate solution, versatic acid and cyclohexane with vigorous stirring at room temperature. After the reaction is finished, the reaction mixture is extracted with distilled water to deprive unreacted material and the cyclohexane phase is then distilled. The process requires the step of extraction with water and is thus complicated. Moreover, it is necessary to prepare neodymium nitrate (Nd(NO₃)) from neodymium oxide in advance.

We have now found that the lanthanide series metal carboxylates can be produced at high yield by directly reacting the lanthanide series metal oxide with carboxylic acid in the presence of plenty of water, separating the oil phase from the aqueous phase of the resulting lanthanide series metal carboxylate solution by simply allowing the solution to settle down, and removing the residual water of the oil phase by distillation. The lanthanide series metal carboxylates can be obtained with high yield and simplified process.

An object of the present invention is to provide a process for the preparation of a lanthanide series metal carboxylate with high yield.

Another object of the present invention is to provide a process for the preparation of a lanthanide series metal carboxylate with simplified steps and fast settlement of the oil phase and aqueous phase so as to shorten the preparation time effectively.

Still another object of the present invention is to provide a method for using the aforementioned lanthanide series metal carboxylate as a catalyst for the polymerization of diene monomers.

Accordingly, the process of the present invention for producing a lanthanide series metal carboxylate which is represented by the formula Ln(RCOO)₃, wherein Ln is a lanthanide series metal element having an atomic number of 57 to 71 and R is a hydrocarbon group having 1 to 19 carbon atoms, comprises the reaction of:
(A) a lanthanide series metal (Ln) compound selected from their oxides and carbonates;
(B) a carboxylic acid having 2 to 20 carbon atoms;
(C) water; and
(D) an inert solvent
   under a reaction temperature of from 0°C to 300°C; wherein the molar ratio of water (C) to the lanthanide series metal (Ln) is greater than 5:1.

The lanthanide series metal carboxylate produced by the process of the present invention is suitable for use as a catalyst for the polymerization of diene monomers to raise the conversion of the monomers.

The lanthanide series metals are elements of the rare earth metals which have atomic numbers of 57 to 71, such as cerium, praseodymium, neodymium, gadolinium, lanthanum and samarium. Examples of the lanthanide series metal compounds (A) suitable for use in the process of the present invention include neodymium oxide, praseodymium oxide, cerium oxide, lanthanum oxide, gadolinium oxide, neodymium carbonate, praseodymium carbonate, cerium carbonate, lanthanum carbonate and gadolinium carbonate. The lanthanide series metal compounds (A) for use in the present invention can be one of the above metal compounds or a mixture thereof. Neodymium oxide is preferred. The selection of the lanthanide series metal oxide is based on the availability of the compound and the activity of the resulting lanthanide carboxylate for use as a catalyst in the polymerization of diene monomers.

The carboxylic acid having 2 to 20 carbon atoms (B) used in the process of the present invention is selected from aliphatic, cycloaliphatic, and aromatic carboxylic acids corresponding to the following formula:

R-COOH

in which
R is C₁₋₁₉ alkyl group, cycloalkyl group, aryl group, or alkylaryl group.

Examples of the carboxylic acid include acetic acid, propionic acid, n-butyric acid, isobutyric acid, valeric acid, pivalic acid, hexane carboxylic acid, 2-hexane carboxylic acid, cyclohexane carboxylic acid, 3-cyclohexyl propionic acid, 2-ethyihexane carboxylic acid, 2-methyl-2-ethyl pentanoic acid, 2,2-dimethyl hexanoic acid, 2-methyl-2-ethyl hexanoic acid, 2,2-diethyl hexanoic acid, 2-ethyl-2-propyl hexanoic acid, 2-ethyl-2-butyl hexanoic acid, 2,2-diethyl heptanoic acid, 2,2-diethyl octanoic acid, 2-methyl-2-butyl octanoic acid, 1-heptanoic acid, 1-octane carboxylic acid, 1-nonane carboxylic acid, versatic acid (i.e., neodecanoic acid having the formula C₉H₁₉COOH), lauric acid, myristic acid, palmitic acid, stearic acid, oleic acid, benzoic acid, naphthenic acid, phenyl acetic acid, triphenyl acetic acid, disproportionated rosin acid and tricyclohexyl acetic acid. Versatic acid and naphthenic acid are preferred. The carboxylic acids can be used individually or in admixture with one another.

The molar ratio of the C₂ to C₂₀ carboxylic acid (B) to the lanthanide series metal (RCOOH/Ln) is in the range of 2/1 to 100/1, preferably 2/1 to 20/1, more preferably 3/1 to 10/1.

The molar ratio of water (C) to the lanthanide series metal (H₂O/Ln) is greater than 5/1. That is, an excessive amount of water with respect to lanthanide series metal must be used in the process of the present invention. If the molar ratio H₂O/Ln is less than 5/1, the reaction would be incomplete with lower product yield, and they are cloudy in both oil and aqueous phase which makes phase separation difficult and time consuming. If necessary, an acid such as hydrogen chloride may be added into the water (C) to facilitate proceeding with the reaction.

Suitable inert solvents (D) for use in the process of the present invention include aliphatic, cycloaliphatic and aromatic hydrocarbons containing 4 to 20 carbon atoms and preferably 4 to 10 carbon atoms, such as butane, pentane, hexane, heptane, octane, cyclohexane, cyclodecane and toluene and their corresponding isomers, preferably n-hexane and cyclohexane. The inert solvents may be used individually or in admixture with one another, which mixing ratio may be determined by a suitable preliminary test.

The process of the present invention may be carried out by introducing the lanthanide series metal compound (A), the carboxylic acid (B) containing 2 to 20 carbon atoms, water (C) and the inert solvent (D), in which the molar ratio of water (C) to the lanthanide series metal (Ln) is greater than 5/1, to a stirring reactor with a reaction time of 0.1 to 8 hours, at a reaction temperature of from 0°C to 300 °C, preferably from 25°C to 160°C. The resulting solution mixture after reaction which contains the metal carboxylate [(Ln(RCOO)₃] is allowed to stand for a time ranging from a few minutes to 2 hours so as to let the two phase solution mixture to settle down and separate into an upper oil phase and a lower aqueous phase. Both oil phase and aqueous phase are clear and the intersurface of them can be seen clearly. The aqueous phase is then removed, and the oil phase which contains the desired lanthanide series metal carboxylate (Ln(RCOO)₃) is distilled until the moisture content thereof is less than 2000 ppm (based on the oil phase).

For use as a catalyst in the polymerization of diene monomers, the moisture content of the lanthanide series metal carboxylate solution produced by the process of the present invention should be not greater than 2000 ppm, preferably not greater than 1500 ppm, more preferably not greater than 1000 ppm. If the moisture content is greater than 2000 ppm, the activity of the catalyst for the polymerization of diene monomers is reduced and results in a lower degree of conversion of the diene monomers.

Examples of the diene monomers used in the present invention are butadiene, isoprene, 2-phenyl butadiene, 2,3-dimethyl butadiene, 1,3-hexadiene and 1,3-octadiene.

The lanthanide series metal carboxylate (1) produced by the process of the present invention can be coordinated together with an alkyl aluminum halide compound (2) and an organo aluminum compound (3) to form a solution of catalyst system for the polymerization of diene monomers.

The alkyl aluminum halide compound (2) that is suitable for use in the catalyst system is selected from the group consisting of compounds represented by the formulae R²AlX₂, R²₃Al₂X₃ and R²₂AlX wherein R² is a hydrocarbon residue having from 1 to 12 carbon atoms; and X is Cl, Br, F or I. Exemplified groups represented by R² includes straight and branched chain aliphatic hydrocarbon groups. Illustrative nonlimiting examples of suitable alkyl aluminum halide compounds (2) include dioctyl aluminum chloride, octyl aluminum sesquichloride, octyl aluminum dichloride, didecyl aluminum chloride, decyl aluminum sesquichloride, decyl aluminum dichloride, didodecyl aluminum chloride, dodecyl aluminum sesquichloride, dodecyl aluminum dichloride, dimethyl aluminum chloride, diethyl aluminum chloride, diethyl aluminum bromide, diethyl aluminum iodide, diethyl aluminum fluoride, di-n-propyl aluminum chloride, di-n-butylaluminum chloride, di-isobutylaluminum chloride (DIBAC), methylaluminum dichloride, ethylaluminum dichloride, isobutylaluminum dichloride, sesquimethylaluminum chloride, sesquiethylaluminum chloride, sesquiisobutylaluminum chloride, and the like, and they may be used alone or in admixture of two or more. The most preferred alkyl aluminum halide compound (2) for use in the catalyst system is di-isobutyl aluminum chloride (DIBAC). The alkyl aluminum halide compound (2) is typically added with a suitable solvent to form a solution of the alkyl aluminum halide compound (2) for use in the catalyst system.

The organo aluminum compound (3) used in the catalyst system is represented by the formula AlR³R⁴R⁵ wherein R³, R⁴ and R⁵ may be identical or different, represented hydrogen atoms or hydrocarbon substituent having 1 to 8 carbon atoms, excluding the case where R³, R⁴ and R⁵ are hydrogen atoms at the same time. Illustrative nonlimiting examples of such compounds include diethyl aluminum hydride, dipropyl aluminum hydride, diisopropyl aluminum hydride, dibutyl aluminum hydride, diisobutyl aluminum hydride (DIBAH), dipentyl aluminum hydride, dihexyl aluminum hydride, trimethyl aluminum, triethyl aluminium, tri-n-propyl aluminum, triisopropyl aluminum, tri-n-butyl aluminum, triisobutyl aluminum, trihexyl aluminum, tricyclohexyl aluminum and the like. They may be used alone or in admixture of two or more. The preferred organo aluminum compound (3) for use in the catalyst system is selected from the group consisting of diethyl aluminum hydride, diisopropyl aluminum hydride and diisobutyl aluminum hydride, diisobutyl aluminum hydride (DIBAH) being the compound that is the most preferred. Besides the above alkyl aluminum halide compound (2) and organo aluminum compound (3), the catalyst system may further contain aluminum halide compound such as aluminum chloride, aluminum bromide and the like.

In order to achieve a high degree of conversion of monomers in the polymerization of the diene monomers, the mixing ratio of the components (1), (2) and (3) in the catalyst system should be limited. The molar ratio of the lanthanide series metal carboxylate (1) to the sum of the alkyl aluminum halide compound (2) and the organo aluminum compound (3) [(1)/(2)+(3)] is in the range of 1:1.5 to 1:100, preferably in the range of 1:1.5 to 1:60. The molar ratio of the lanthanide series metal carboxylate (1) to the alkyl aluminum halide compound (2) [(1)/(2)] is in the range of 1:0.5 to 1:10, preferably 1:1 to 1:5. The molar ratio of the lanthanide series metal carboxylate compound (1) to the organo aluminum compound (3) [(1)/(3)] is in the range of 1:1 to 1:50, preferably in the range of 1:2 to 1:20.

A suitable inert hydrocarbon could be used in the polymerization. Suitable inert hydrocarbons for use as the polymerization medium include aliphatic, cycloaliphatic, aromatic and monoolefinic hydrocarbons and mixtures thereof. More specifically, suitable inert hydrocarbons are those selected from the group consisting of C₄ to C₈ aliphatic hydrocarbons, C₅ to C₁₀ cyclic aliphatic hydrocarbons, C₆ to C₉ aromatic hydrocarbons, C₄ to C₈ monoolefinic hydrocarbons and mixtures thereof. Illustrative nonlimiting examples of the aforementioned hydrocarbons include butane, pentane, hexane, heptane, cyclopentane, cyclohexane, benzene, toluene, xylene, butene-1 and pentene-1. Preferably the polymerization process is carried out in a polymerization medium that does not contain an aromatic hydrocarbon. That is, the process is carried out in hydrocarbons selected from the group consisting of C₄ to C₈ aliphatic hydrocarbons, C₅ to C₁₀ cyclic aliphatic hydrocarbons, and C₄ to C₈ monoolefinic hydrocarbons and mixtures thereof as the catalyst system exhibits an improved activity in such a polymerization medium. More preferably, the inert hydrocarbon for use as the polymerization medium is selected from hexane and cyclohexane.

To prepare the catalyst system, solutions of components (1), (2) and (3) may be mixed in any desired sequence in a suitable solvent with stirring. The temperature at which preparation of the catalyst system is carried out may vary within a wide range and is generally limited by the melting point and the boiling point of the solvent used. Temperatures ranging from -20°C to 120°C are suitable. Preparation of the catalyst system may be carried out separately or, preferably, by the addition and mixing of the organo aluminum compound (3) and the lanthanide series metal carboxylate (1) with the polymerization reaction mixture followed by the addition of the alkyl aluminum halide compound (2). If desired, organo aluminum compound (3) and the lanthanide series metal carboxylate (1) may be mixed together before they are added to the polymerization mixture of diene monomers.

In preparing the catalyst system, a suitable conjugated diene may be added to improve the polymerization activity of the catalyst system and to shorten the induction time for the initiation of polymerization. The conjugated diene may be added at any time in each of the above-mentioned order of additions. The molar ratio of the lanthanide series metal carboxylate to the conjugated diene is 1:0 to 1:1000, preferably 1:0.5 to 1:500, more preferably 1:2 to 1:100. Examples of the conjugated diene are isoprene, butadiene, 1,3 -pentadiene.

During polymerization, the conjugated diene monomers may be added before or after the catalyst components or between the addition of one catalyst component and the addition of another catalyst component. The conjugated diene monomers may be added at one time or incrementally. Polymerization is conducted in one reactor or a series of reactors provided with a stirrer. When the polymerization has reached a predetermined degree of conversion, a deactivate agent, such as water and alcohol, is added for removal, of residual catalyst activity. The polymerization mixture is then evaporated and dried to produce the polybutadiene rubber. The butadiene polymerization reaction catalyzed by using the lanthanide series metal carboxylate which was prepared according to the process of the present invention can produce a high content of 1,4-cis-polybutadiene rubber. Typically, the content of 1,4-cis configuration is greater than 95%.

The present invention is more specifically described and explained through the following Examples and Comparative Examples. Examples 1 to 7 illustrate the process of the present invention for producing the lanthanide series metal carboxylate. Examples 8 and 9 illustrate polymerization of diene monomers using the lanthanide series metal carboxylates produced by the process of the present invention.

### [Example 1]

The reaction mixture of 0.500kg (1.46mol) neodymium oxide (Nd₂O₃, 99% purity), 2.100 kg (12.16 mols), and versatic acid (a commercial product of Shell chemicals, having an acid value of 324) is well mixed, and then added with 3.700 kg(42.67mols) n-hexane and 2.100 kg (116.7 mols) water. The resulting solution mixture is fed into a 20 liters reactor. The reaction is conducted at 95°C for 2.5 hours. Thereafter, the reacted solution mixture is allowed to stand for 30 minutes to permit the separation of an upper oil phase and a lower aqueous phase. The bottom of the reactor is free of any precipitate of neodymium oxide. Both the upper oil phase and the lower aqueous phase are clear. The lower aqueous phase is removed. The remaining upper oil phase that contains Nd(versatate)₃ and n-hexane is distilled by a distillator to produce a solution of Nd(versatate)₃. The solution of Nd(versatate)₃ thus obtained has a moisture content of 120 ppm and the yield of Nd(versatate)₃ is 99.5%.

### [Example 2]

The reaction of Example 1 is repeated with the exception that the amount of water used is decreased to 1.050 kg (58.3 mols). The reacted solution mixture is allowed to stand for 60 minutes after the reaction is finished to permit the separation of a clear upper oil phase and a clear lower aqueous phase. The bottom of the reactor is free of any precipitate of neodymium oxide. The solution of Nd(versatate)₃ thus obtained has a moisture content of 220 ppm after distillation and the yield of Nd(versatate)₃ is 95%.

### [Example 3]

The reaction of Example 1 is repeated with the exception that the amount of water used is increased to 3.47 kg (193 mols). Thus, the molar ratio of water to the lanthanide series metal (H₂O/Ln) becomes 66/1. The reacted solution mixture is allowed to stand for 30 minutes after the reaction is finished to permit the separation of a clear upper oil phase and a clear lower aqueous phase. There is no precipitate of neodymium oxide at the bottom of the reactor. The solution of Nd(versatate)₃ thus obtained has a moisture content of 280 ppm after distillation, and the yield of Nd(versatate)₃ is 99.5%.

### [Example 4]

The reaction of Example 1 is repeated with the exception that the amount of versatic acid used is 1.44 kg (8.76 mols). Similarly, the reacted solution mixture is allowed to stand for 30 minutes after the reaction is finished to permit separation of a clear upper oil phase and a clear lower aqueous phase. There is no precipitate of neodymium oxide at the bottom of the reaction tank. The solution of Nd(versatate)₃ thus obtained has a moisture content of 150 ppm after distillation and the yield of Nd(versatate)₃ is 98%.

### [Example 5]

The reaction of Example 1 is repeated with the exception that the amount of versatic acid used is increased to 3.000 kg (8.76 mols). Thus, the molar ratio of versatic acid to the neodymium oxide becomes 6/1. Likewise, the reacted solution moisture is allowed to stand for 30 minutes after the reaction is finished to permit separation of a clear upper oil phase and a clear lower aqueous phase. The reactor is free of any precipitate of neodymium oxide at the bottom thereof. The solution of Nd(versatate)₃ thus obtained has a moisture content of 450 ppm after distillation and the yield of Nd(versatate)₃ is 99.5%.

### [Example 6]

The reaction of Example 1 is repeated with the exception that 10 ml hydrogen chloride solution (35.5%) is further added into the reactor tank and that the reaction is conducted at a temperature of 80°C for 4.0 hours. The reacted solution mixture is allowed to stand for 60 minutes after the reaction is finished to permit separation of a clear upper oil phase and a clear lower aqueous phase. There is no precipitate of neodymium oxide at the bottom of the reaction tank. The solution of Nd(versatate)₃ thus obtained has a moisture content of 500 ppm after distillation and the yield of Nd(versatate)₃ is 99%.

### [Example 7]

0.170kg (0.5 mol) neodymium oxide, 0.760 kg (4.07 mols) naphthenic acid with an acid value of 300, 3.400 kg (43.7 mols) n-hexane and 1.410 kg (78.3 mols) water are fed into a 20 liters reactor. The reaction is conducted at 150°C for 3 hours with stirring. After the reaction is finished, the reacted solution mixture is allowed to stand for 1.5 hours to permit separation of an upper oil phase and a lower aqueous phase. The lower aqueous phase is removed, and the upper oil phase containing Nd(Naph)₃ and n-hexane is distilled. The solution of Nd(Naph)₃ thus obtained has a moisture content of 500 ppm, and the yield of Nd(Naph)₃ is 90%.

### [Comparative Example 1]

The reaction in Example 1 is repeated with the exception that the amount of water is decreased to 0.017kg (0.9 mol). Thus, the molar ratio of water to the lanthanide series metal (H₂O/Ln) becomes 0.31/1. After 2.5 hours of reaction, the reacted solution mixture is allowed to stand for 15 hours to permit separation of an upper oil phase and a lower aqueous phase. However, both the oil phase and the aqueous phase are cloudy even after the prolonged separation time, and precipitate of Nd₂O₃ appears in the bottom of the reactor, indicating that the reaction is incomplete. For the purpose of removing the suspended Nd₂O₃ or other side products which make the oil phase cloudy, it is necessary to treat the oil phase by high-speed centrifuge to isolate those suspended materials. The oil phase is then distilled for removal of water to provide the Nd(versatate)₃ solution. The Nd(versatate)₃ solution thus obtained has a moisture content of 280 ppm and the yield of Nd(versatate)₃ is 78%.

### [Comparative Example 2]

The reaction of Example 4 is conducted with the exception that the amount of water is decreased to 0.0072 kg (0.4 mol). Thus, the molar ratio of water to the lanthanide series metal (H₂O/Ln) becomes 0.14/1. After the same separating procedures, even when the separation time is increased to 12 hours, the upper oil phase and the lower aqueous phase are still cloudy. Also, precipitate of Nd₂O₃ appears in the bottom of the reactor. For the purpose of removing the Nd₂O₃ suspended materials in the oil phase, it is necessary to treat the oil phase by high-speed centrifuge to result in a solution of Nd(versatate)₃ having a moisture content of 10200 ppm without distillation and the yield of Nd(versatate)₃ is 45%.

The solution of Nd(versatate)₃ thus obtained is then used as a catalyst for the polymerization of butadiene monomers, the same procedures as described in Example 8. It is found that the polymerization cannot be proceeded due to the high moisture content of the catalyst.

### [Example 8]

14.900 kg of the solution of Nd(versatate)₃ (containing 0.256 mol Nd(versatate)₃) prepared in Example 1 is mixed with 66.270 kg di-isobutyl aluminum hydride (DIBAH)/n-hexane solution (containing 83.6 mols DIBAH) and 20.54 kg di-isobutyl aluminum chloride (DIBAC)/n-hexane solution (containing 20.9 mols DIBAC) with stirring to prepare a solution of the catalyst system.

10.73 kg (198 mols) of 1,3-butadiene monomers which have been previously purified by distillation to have a moisture content of below 2 ppm, are placed in a 200L reactor that is equipped with a stirrer and a cooling system. 82.23 kg (954 mols) n-hexane and 0.678 kg of the solution of the catalyst system are then introduced into the reactor. The n-hexane used in the preparation of catalyst system and in the polymerization have both been distilled previously to have a moisture content of below 2 ppm. The reaction is conducted at a temperature of 90°C with stirring for 100 minutes. After the reaction is finished, the reacted solution is evaporated and dried. The rubbery product thus obtained is polybutadiene which has 98% cis-1,4 configuration. The conversion of butadiene monomers is 95%. Moony viscosity of the polybutadiene is 40.

### [Example 9]

40.27 kg of the solution of Nd(versatate)₃ (containing 0.7 mol Nd(versatate)₃) prepared in Example 4 is mixed with 55.27 kg di-isobutyl aluminum hydride (DIBAH) solution (containing 70 mols DIBAH) and 20.54 kg di-isobutyl aluminum chloride (DIBAC)/n-hexane solution (containing 20.9 mols DIBAC) with stirring to prepare a solution of the catalyst system.

10.73 kg (198 mols) of 1,3-butadiene monomers are placed in a 200L reactor that is equipped with a stirrer and a cooling system. 82.23 kg (954 mols) n-hexane and 0.71 kg of the solution of the catalyst system are then introduced into the reactor. The butadiene monomers and the n-hexane solvent used in the preparation of catalyst system and in the polymerization have been previously distilled to have a moisture content of below 2 ppm. The reaction is conducted at a temperature of 95°C with stirring for 110 minutes. After the reaction is finished, the reaction solution is distilled and dried. The rubbery polymer thus obtained is polybutadiene which has 98% cis-1,4 configuration. The conversion of butadiene monomers is 94%. Moony viscosity of the polybutadiene is 42.

The reaction components, the amounts thereof, the reaction conditions and the properties of the products of Examples 1 to 7 and Comparative Examples 1 and 2 are listed in Table 1.

In light of the foregoing Examples and Comparative Examples, it is found that when an excessive amount of water with respect to lanthanide series metal (Ln) is added into the lanthanide series metal oxide in the process of the present invention such that the molar ratio of water (C) to the lanthanide series metal (Ln) is greater than 5/1, the reaction is completed and the oil phase and the aqueous phase are clear and can be separated from one another within a relatively short time. An additional centrifugal isolating step of the oil phase can thus be obviated. The process of the present invention involves relatively simple steps and produces the lanthanide series metal carboxylate product at high yield. When the lanthanide series metal carboxylate which is prepared by the process of the present invention is used as a catalyst for the polymerization of diene monomers, the reaction rate of the polymerization process can be significantly raised.

With this invention thus explained, it is apparent that numerous modifications and variations can be made without departing from the scope and spirit of this invention. It is therefore intended that this invention be limited only as indicated in the appended claims.

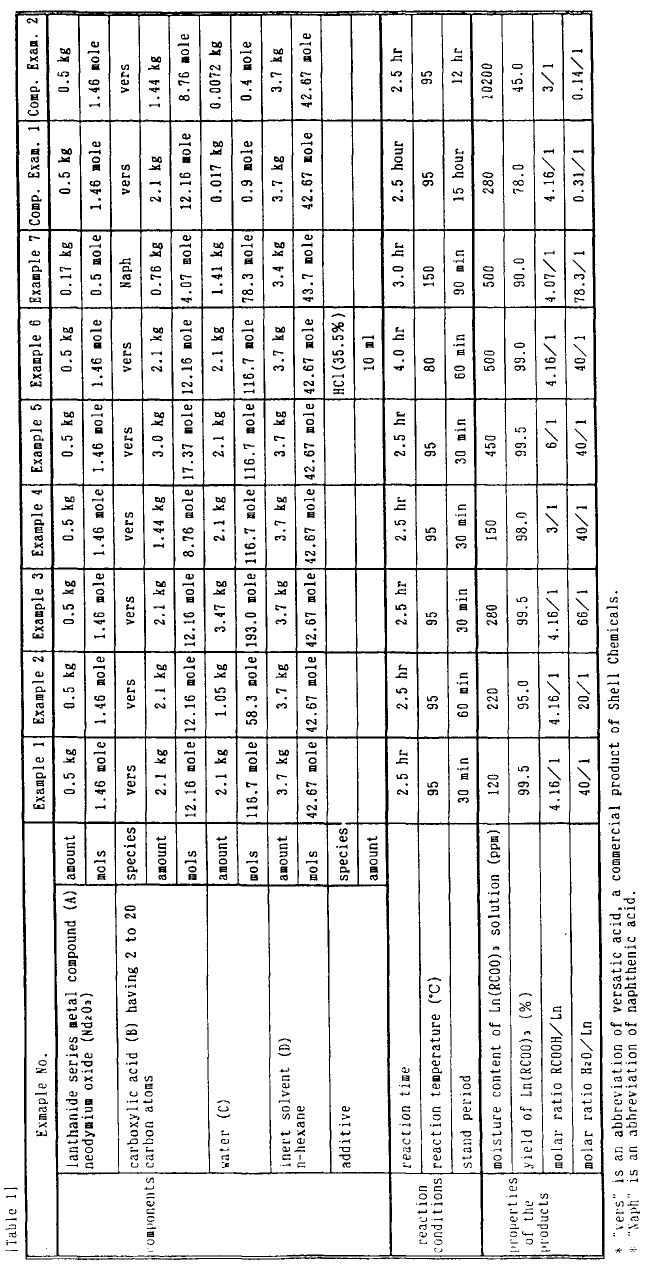

## Claims

1. A process for producing a compound of formula Ln(RCOO)₃, wherein Ln is a lanthanide series metal element of atomic number 57 to 71 and R is a C₁-C₁₉ hydrocarbon group, which process comprises reacting together:
(A) an oxide or carbonate of a lanthanide series metal Ln as defined above;
(B) a C₂-C₂₀ carboxylic acid;
(C) water; and
(D) an inert solvent
at a temperature of from 0°C to 300°C, wherein the molar ratio of water to the lanthanide series metal is at least 5:1.

2. A process according to claim 1, wherein the molar ratio of water to the lanthanide series metal is from 5:1 to 200:1.

3. A process according to claim 1 or 2, wherein the molar ratio of water to the lanthanide series metal is from 15:1 to 80:1.

4. A process according to any one of the preceding claims which further comprises isolating the oil phase from the aqueous phase of the reaction product after the reaction is finished, and distilling the oil phase such that the oil phase has a moisture content of 2000 ppm or less (based on the oil phase).

5. A process according of claim 4, wherein the step of isolating the oil phase from the aqueous phase is completed within 2 hours.

6. A process according to any one of the preceding claims wherein the lanthanide series metal is neodymium.

7. A process according to any one of the preceding claims wherein the carboxylic acid is naphthenic acid or versatic acid.

8. A process according to any one of the preceding claims wherein the molar ratio of the carboxylic acid to the lanthanide series metal is from 2:1 to 100:1.

9. A process according to any one the preceding claims, wherein the molar ratio of the carboxylic acid to the lanthanide series metal is from 3:1 to 10:1.

10. A process for producing a polymer derived from diene monomers, which process comprises polymerizing diene monomers in the presence of a compound of formula Ln(RCOO)₃ which is obtainable by the process defined in any one of claims 1 to 9.
